# EUROPÄISCHE PATENTSCHRIFT

(11) **EP 0 876 311 B1**
(45) Veröffentlichungstag und Bekanntmachung des Hinweises auf die Patenterteilung: **18.08.1999**
(21) Anmeldenummer: 97901083.2
(22) Anmeldetag: 24.01.1997
(51) Int. Cl.: C05F 17/02

(54) **VERFAHREN ZUR BIOLOGISCHEN BEHANDLUNG VON ORGANISCHEN MATERIALIEN UND VORRICHTUNG ZUR DURCHFÜHRUNG DES VERFAHRENS**
PROCESS FOR BIOLOGICALLY TREATING ORGANIC MATERIALS AND DEVICE FOR CARRYING OUT THIS PROCESS
PROCEDE POUR LE TRAITEMENT BIOLOGIQUE DE MATIERES ORGANIQUES ET DISPOSITIF POUR LA MISE EN OEUVRE DU PROCEDE

(30) Priorität: 25.01.1996 DE 19602489
(43) Veröffentlichungstag der Anmeldung: 11.11.1998
(73) Patentinhaber: Widmer, Christian, 4052 Basel (CH)
(72) Erfinder: WIDMER, Christian, CH-4052 Basel (CH); STEHLE, Wolfgang, D-88281 Schlier (DE); WELLINGER, Artur, CH-8357 Guntershausen (CH)
(74) Vertreter: Otten, Herbert
(86) Internationale Anmeldenummer: EP9700341
(87) Internationale Veröffentlichungsnummer: WO9727158

(56) Entgegenhaltungen:
- EP-A- 0 567 184
- WO-A-95/20554
- DE-A- 4 423 099
- DE-C- 4 343 767
- US-A- 5 288 399

## Beschreibung

Die Erfindung betrifft ein Verfahren zur biologischen Behandlung von organischen Materialien nach dem Oberbegriff des Anspruchs 1. Gleichermaßen betrifft die Erfindung eine zugehörige Vorrichtung zur Durchführung des Verfahrens.

### Stand der Technik:

Die Abfallwirtschaft befindet sich seit einigen Jahren in einem beschleunigten Umstrukturierungsprozeß. Dabei tritt auch das Problem der Entsorgung und Verwertung von biologischen Abfällen aus Haushalt, Gewerbe und Industrie immer mehr in den Vordergrund. Es sind zahlreiche Verfahren zur biologischen, mechanisch-biologischen oder chemischbiologischen Restabfallverwertung bekannt geworden. Als bekanntestes Verfahren wird die Kompostierung von tierischen und pflanzlichen Abfällen verwendet, wobei die organischen Substanzen in einem aeroben Umsetzungsprozeß mittels Mikroorganismen weitgehend ab- oder umgebaut werden. Neben dem aerobe Verrottungsprozeß ist auch die anaerobe Vergärung zur Behandlung biologischer Abfälle bekannt geworden, die unter Luftabschluß ebenfalls unter Einwirkung von Mikroorganismen eine Müllzersetzung bewirkt. Dabei werden beide Verfahren einzeln oder auch kombiniert in der Abfalltechnik verwendet.

Insbesondere bei der Kompostierung mit einer aeroben Behandlung wird in einem bekannten Vorrotteverfahren das Material zu seiner Durchmischung in einer Rottetrommel dauernd bewegt und in einer Zeitdauer von ca. ein bis zwei Tagen zu Frischkompost umgesetzt. Bei einer statischen Vorrotte ruht das zu kompostierende Material und wird hierbei belüftet. Dies erfordert insbesondere auch für den nachfolgenden Nachrottevorgang einen erheblichen Platzbedarf. Der Vorteil der dynamischen Vorrotte mit einer ständigen Durchmischung des Müllgemisches liegt in der guten Belüftung des Materials, wobei das Auftreten von anaeroben Stellen im Material verhindert wird.

Die in den letzten Jahren ebenfalls bekannt gewordene anerobe Behandlung organischer Müllabfälle oder Bioabfälle beruht auf einer anaeroben Fermentation, d. h. auf einem Faulungsprozeß, der unter Luftabschluß bei schwach exotermer Reaktion zu einer Biogas-Produktion führt. Ein solches Verfahren ist beispielsweise aus der EP 0 037 612 B1 bekannt geworden, bei welchem in einem stationären Verfahren organische Abfälle in einem Reaktor mit einer Auswaschflüssigkeit behandelt werden, welches lösliche anorganische und/oder organische Materialien aus dem zu behandelnden Material auswäscht. Dabei findet dieser Prozeß unter Luftabschluß, d. h. unter anaeroben Bedingungen als sogenannte Hydrolyse statt.

Nachteilig an diesem bekannten Verfahren ist aufgrund der stationären Anordnung des Materials die Entstehung von Kurzschlußstromkanälen, d. h. die aufgegebene Flüssigkeit sucht sich über einzelne Kanäle den Weg des geringsten Widerstands aufgrund einer Art Kaminwirkung, so daß das Material nur unvollständig mit Waschflüssigkeit berieselt wird. Das Ergebnis sind Totzonen, welche nicht, oder nur ungenügend ausgewaschen werden.

Aus der WO-A-95/20 554 ist ein Verfahren zur biologischen Behandlung von organischen Abfällen bekannt geworden, bei welchem leicht abbaubare organische Abfallstoffe ausschließlich mittels eines aeroben Prozesses zu Kompost umgesetzt werden sollen. Dabei erfolgt eine Reduktion der organischen Substanz durch einen biologischen Abbau. Hierzu wird Luft vom oberen Luftraum mittels Ventilatoren nach unten durch das Material geführt und mittels Unterdruck abgesaugt. Um den biologischen Abbauprozeß zu beschleunigen, wird das Material mit verschieden Rührwerken an einer vorbestimmten Stelle quer zu Laufrichtung aufgelockert und gegebenenfalls mit einem Wasservorhang befeuchtet. Die mit Abfall beladenen Tablare werden dann mit einem hydraulischen Stößel schrittweise durch den Komposter geschoben, hinten entnommen und gegebenenfalls vorne wieder eingetragen.

### Aufgabe und Vorteile der Erfindung:

Der Erfindung liegt die Aufgabe zugrunde, ein verbessertes Verfahren zur biologischen Behandlung insbesondere von nassen organischen Materialien (Biomüll) vorzuschlagen, bei welchem die vorgenannten Nachteile nicht auftreten und welches insbesondere eine äußerst effektive und kostengünstige Behandlungsmethode für derartige Materialien darstellt. Dabei liegt der Erfindung der Kerngedanke zugrunde, daß eine kombinierte Behandlung des Materials in Form eines Auswaschprozesses und einer gezielten aeroben Materialbehandlung zu einer höchst effektiven Verrottung des Produkts führt. Dabei bedient sich die Erfindung einzelner, zum Teil an sich bekannter Verfahrensschritte, die jedoch in ihrer Kombination zu einer effektiven Behandlung des Biomülls führen.

Das erfindungsgemäße Verfahren hat insbesondere den Vorteil, daß aufgrund einer dynamischen Behandlung des Materials Kurzschlußstromkanäle sowohl in vertikaler als auch in horizontaler Richtung im Material verhindert oder zerstört werden, so daß die durchgeführte Berieselung mit Auswaschflüssigkeit das Material gleichmäßig und überall erfaßt, so daß sich keine Totzonen bilden. Die organische Masse wird kontinuierlich oder diskontinuierlich umgewälzt und gleichzeitig einer aeroben Materialzersetzung unterzogen. Dabei erfolgt die Berieselung von oben nach unten unter zeitweise gleichzeitiger Beaufschlagung mit Luft von unten nach oben zur Erzielung eines aeroben Abbaus.

In einem erfindungsgemäß ausgebildeten Reaktor mit einer entsprechenden Umwälzeinrichtung für das Material sowie einer Berieselungsanlage und Mittel zur Zuführung von Frischluft und Abführung der Auswaschflüssigkeit wird der Verrottungsprozeß durchgeführt.

In den Unteransprüchen sind vorteilhafte und zweckmäßige Weiterbildungen des erfindungsgemäßen Verfahrens angegeben.

Besonders vorteilhaft ist der Betrieb des Reaktors im Durchlaufbetrieb, d. h. der Reaktor wird auf einer Seite mit Material beschickt, welches aufgrund des Umwälzwerkes langsam durch den Reaktor transportiert wird. Auf der anderen Seite wird das Material entsprechend aus dem Reaktor entnommen.

Ein solcher Durchlaufbetrieb hat insbesondere den Vorteil, daß über die Länge des Reaktors unterschiedliche Materialeigenschaften bestehen, da aufgrund der Verweildauer von mehreren Tagen, sich der Zersetzungszustand des Materials über die Länge des Reaktors verändert und dementsprechend einer unterschiedlichen Behandlung unterzogen werden kann. Hierdurch erfolgt insbesondere über die Länge des Reaktors eine örtliche und zeitliche unterschiedliche Behandlung sowohl mit Auswaschflüssigkeit als auch mit Frischluft für die aerobe Verrottung.

Der erfindungsgemäß Reaktor ermöglicht deshalb eine äußerst flexible Handhabung des einzubringenden Materials je nach dessen Zusammensetzung und insbesondere je nach organischer Belastung, Temperatur sowie Wassergehalt, wobei insbesondere die aerobe Umsetzung des Materials über die Länge des Reaktors meßtechnisch erfaßt und der Prozeß geregelt oder gesteuert werden kann.

Der Grad der Befüllung des Reaktors mit zu behandelndem Material sowie seine geometrische Formgebung und die Berieselung der Oberfläche des Materials erfolgt vorteilhafterweise derart aufeinander abgestimmt, daß eine gleichmäßige Berieselung der maximal möglichen Materialoberfläche erfolgt, damit sich keine toten Zonen bilden.

Vorteilhaft ist weiterhin, daß im unteren Bereich des Reaktors mehrere Kammern vorgesehen sind, die gleichermaßen zur Zuführung von Frischluft und/oder als Ablauf für die Auswaschflüssigkeit dienen, wobei die Kammern insbesondere durch ein Siebfilter zur weitestgehenden Vermeidung eines Durchtritts von Material abgedeckt sind. Das Abdecksieb kann durch eine Rückspülung gereinigt werden, wobei mehrere Kammern zusammenwirken können.

Vorteilhaft kann auch eine Flutung des unteren Bereichs des Reaktors mit Auswaschflüssigkeit sein, um eine Art Aufschwimmen des Materials für eine bessere Umwälzung zu erhalten, was vorzugsweise bei sehr zähem und/oder sehr trockenem Material zweckmäßig ist.

Das erfindungsgemäße Verfahren wird vorzugsweise dadurch weitergebildet, daß dem Durchlaufreaktor verschiedene mechanisch-biologische Stufe nachgeordnet sind, die eine Reinigung und Regenerierung der Auswaschflüssigkeit vornehmen und diese durch eine anaerobe Behandlung von der starken organischen Beladung befreien. Im Kreislauf kann eine so behandelte Auswaschflüssigkeit dem erfindungsgemäßen Reaktor wieder zugeführt werden.

Vorteile hinsichtlich der erfindungsgemäßen Vorrichtung zur Durchführung des Verfahrens sind in der nachfolgenden Beschreibung eines Ausführungsbeispiels ergänzend angegeben.

Es zeigen
- Fig. 1: eine Seitenansicht eines erfindungsgemäßen Reaktors zur Behandlung des Biomülls mit nachgeordneten Stufen in schematischer Darstellung zur Behandlung der Auswaschflüssigkeit,
- Fig. 2 bis 6: einen Längsschnitt durch den Reaktor mit unten liegender Kammer in verschiedenen Betriebszuständen,
- Fig. 7: einen Ausschnitt auf die Längsansicht der Rührwelle.

### Beschreibung der Erfindung:

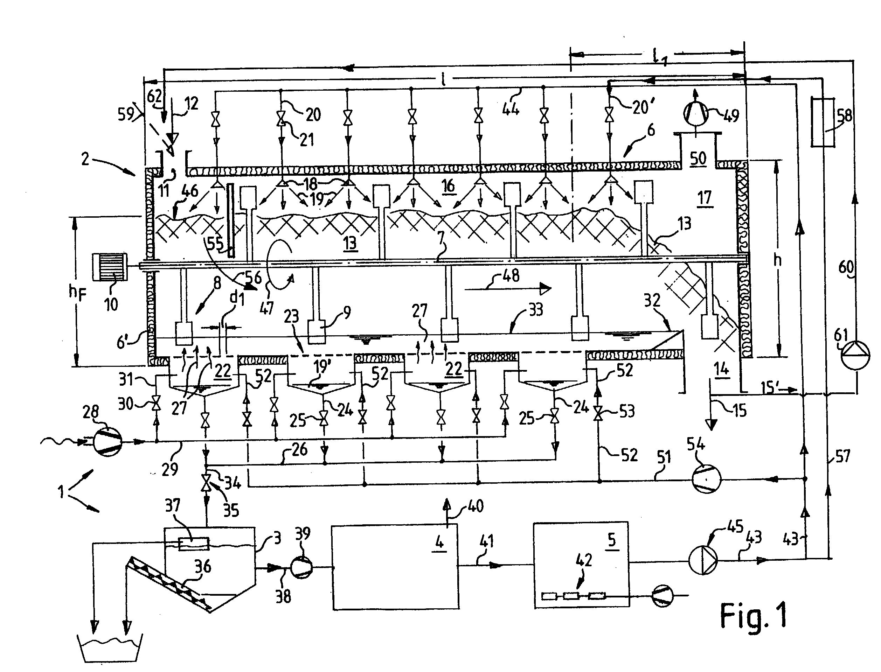

Das in der Fig. 1 dargestellte Prinzipschaubild zeigt eine Anlage 1 zur biologischen Behandlung von vorzugsweise nassem Biomüll mit mehreren Komponenten, bestehend aus einem Feststoffreaktor 2 zur Behandlung des organischen Materials sowie dem Reaktor 2 nachgeschaltete Bearbeitungsstufen 3 bis 5 zur Nachbehandlung und Aufbereitung der wieder verwendbaren Auswaschflüssigkeit.

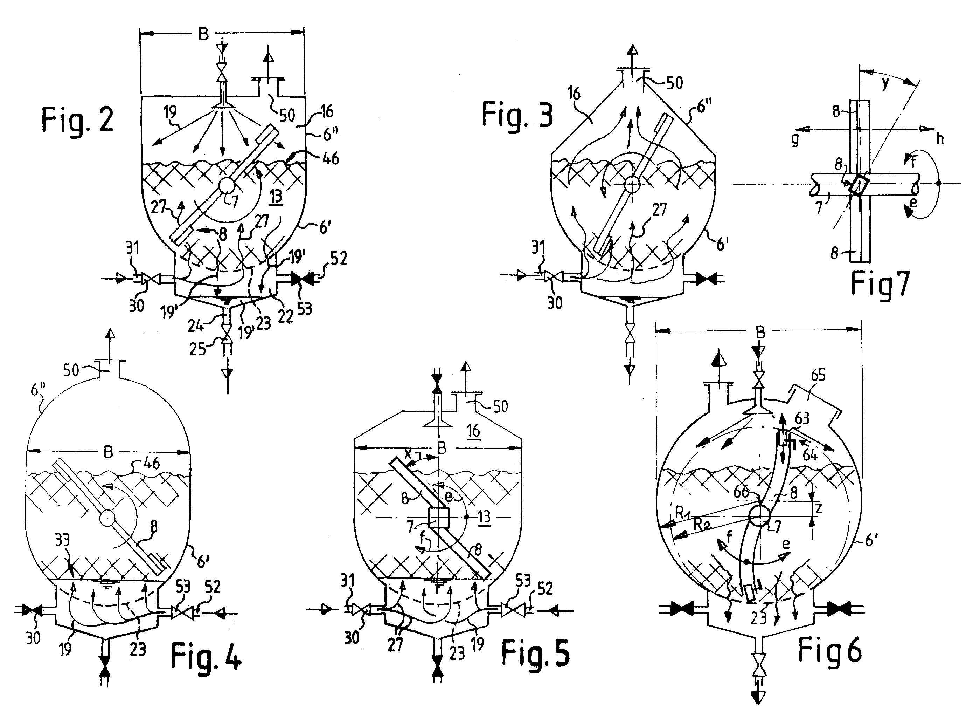

Der Reaktor 2 besteht entsprechend der Darstellung in Fig. 1 aus einem länglichen Gehäuse 6 mit einem vertikalen Querschnitt, wie er in den Figuren 2 bis 5 gezeigt ist. Die Länge 1 des Gehäuses kann je nach Größe der Anlage sehr unterschiedlich ausgestaltet sein und durchaus beispielsweise 1 20 bis 30 m betragen. Die Höhe h des Reaktorraumes beträgt in diesem Fall h 4 bis 5 m. Im unteren Bereich ist das Reaktorgehäuse 6' im Querschnitt etwa zylindrisch, im oberen Bereich 6" eher rechteckförmig (Fig. 2), dreieckförmig (Fig. 3) oder insgesamt oval (Fig. 4) ausgebildet. Das Reaktorgehäuse 6 wird von einer horizontalen Welle 7 durchsetzt, welches vorzugsweise als Stachelrührwerk 8 mit paddel- oder schaufelförmigen Umwälzorganen 9 ausgebildet ist. Der Antrieb der Welle 7 erfolgt über einen Antriebsmotor 10.

Gemäß der Darstellung in Fig. 1 weist das Gehäuse 6 des Reaktors 2 an seinem einen, in der Figur linken oberen Ende eine Einfüllöffnung 11 auf, durch die der Reaktor entsprechend dem Pfeil 12 mit einem organisch belasteten Material 13 beschickt wird. Hierbei handelt es sich insbesondere um nasse organische Materialien oder Biomassen, die insbesondere von biologischen Abfällen im Haushalt aus dem lebensmittelverarbeitenden Gewerbe, der Landwirtschaft oder aus einer organischen Fraktion des Restmülls (Deponiemüll) oder dergleichen (Biomüll) stammen. Dabei kommen die verschiedensten Zusammensetzungen sowohl hinsichtlich der organischen Belastung als auch hinsichtlich des Feuchtigkeitsgrades sowie der Ausgangstemperatur in Betracht.

Am anderen Ende des Reaktors 2 befindet sich gemäß der Darstellung in Fig. 1 im unteren rechten Ende eine Auslaßöffnung 14 für das Material 13 nach erfolgter Behandlung, welches gemäß Pfeil 15 aus dem Reaktor ausgetragen wird.

Der in Fig. 1 dargestellte Reaktor 2 ist in seinem Innenraum 17 bis zu einer mittleren Füllhöhe h_{F} mit Material 13 befüllt, so daß sich ein darüberliegender Luftraum 16 einstellt. Die Füllhöhe des Materials kann beispielsweise zwei Drittel der Gesamthöhe h betragen.

Im oberen Bereich des Reaktorinnenraumes 17 befinden sich eine Vielzahl von Sprüharmen 18 zur Beaufschlagung des Materials 13 mit einer Auswaschflüssigkeit 19, die gemäß einer Pfeildarstellung aus den Sprüharmen 18 heraustritt. Eine obere Zuleitung 20 mit Steuerungsventil 21 führt die Auswaschflüssigkeit 19 zu den einzelnen Sprüharmen 18. Im Ausführungsbeispiel nach Fig. 1 sind beispielsweise sieben Sprüharme 18 über die Länge des Reaktorinnenraums verteilt angeordnet.

Im unteren Bereich des Reaktors 2 sind im Ausführungsbeispiel nach Fig. 1 mehrere Kammern 22 und insbesondere vier Kammern 22 nebeneinanderliegend angeordnet, die mittels eines Abdecksiebes 23 gegenüber dem Innenraum 17 des Reaktors abgetrennt sind, damit das Material 13 möglichst nicht in die Kammer 22 fällt. Der Lochsiebdurchmesser liegt zwischen 6 bis 12 mm und beträgt insbesondere 8 mm Durchmesser. Dieser Durchmesser ist mit d₁ symbolisch dargestellt.

Die Kammer 22 hat eine Doppelfunktion. Sie dient einerseits zur Sammlung der durch das Material 13 hindurchtretenden Auswaschflüssigkeit. Diese Auswaschflüssigkeit ist mit 19' in der Kammer 22 gekennzeichnet und wird über eine Abfuhrleitung 24 mit einer Ventilanordnung 25 zu einer Sammelleitung 26 geführt.

Zusätzlich dient die Kammer 22 zur Beaufschlagung des Materials 13 mit Frischluft 27, wie sie symbolisch in Fig. 1 mit Pfeilen dargestellt ist. Die Frischluft wird über einen Kompresser 28 einer Sammelleitung 29 zugeführt, und von dort über Steuerventile 30 über die Leitung 31 der jeweiligen Kammer 22 zugeführt.

Aufgrund der vorgesehenen Möglichkeit zur Rückspülung und Beflutung des Reaktorinnenraums 17 mit Auswaschflüssigkeit aus den Kammern 22 ist eine Überlaufwandung 32 oder Überlaufkante im Bereich der Auslaßöffnung 14 vorgesehen. Der zugehörige obere Flüssigkeitsspiegel 33 ist symbolisch dargestellt.

Die untere Sammelleitung 26 für die aus den Kammern abgeführte Auswaschflüssigkeit führt über eine gemeinsame Leitung 34 mit Ventilanordnung 35 zu den nachgeschalteten Bearbeitungsstufen 3 bis 5 zur Behandlung der Auswaschflüssigkeit. Diese Behandlungsstufen umfassen insbesondere zunächst einen Störstoffabscheider 3, der zur Abscheidung von Sinkstoffen aller Art wie z. B. Sand, Steine oder dergleichen dient, d. h. alle Stoffe, die als Sinkstoffe das Sieb 23 durchdringen. Absetzstoffe werden über eine Fördereinrichtung 36, Schwimmstoffe oder Schwebstoffe wie Kunststoff, Holz oder dergleichen werden über einer Skimmereinrichtung 37 aus dem Störstoffabscheider entfernt.

Die so im Störstoffabscheider 3 gereinigte Auswaschflüssigkeit wird über eine Leitung 38 mit Pumpe 39 zu einer nachgeschalteten anaeroben Reaktorstufe 4 geführt, der beispielsweise als Metanreaktor ausgebildet ist. In diesem Metanreaktor wird die mit Organik angereicherte Auswaschflüssigkeit durch Metanbakterien abgebaut und gereinigt, wobei Biogas oder Metangas 40 als Stoffwechselprodukt entsteht.

Schließlich enthält die nachgeschaltete Behandlungsstufe einen weiteren aeroben Reinigungsreaktor 5, der über eine Leitung 41 den anaeroben Zustand der Auswaschflüssigkeit in einen aeroben Zustand überführt, wobei eine Belüftung 42 für eine aerobe Zersetzung und Belebung der Auswaschflüssigkeit sorgt.

Für diese drei nachgeschalteten Behandlungsstufen 3 bis 5 für die Auswaschflüssigkeit können herkömmliche Bauelemente und Anlagenteile nach dem bekannten Verfahrensschema verwendet werden.

Die so behandelte und gereinigte und insbesondere von starker organischer Belastung entsorgte Auswaschflüssigkeit wird über eine Leitung 43 der aeroben Reinigungsstufe 5 entnommen und einer Sammelleitung 44 mittels einer Pumpe 45 zugeführt. Diese Sammelleitung 44 führt zu den einzelnen Zuleitungen 20 zu den Sprüharmen 18.

Eine derartig behandelte Auswaschflüssigkeit besteht nach einer bestimmten Anlaufphase der Anlage 1 aus einer leicht versäuerten Flüssigkeit, wobei die Versauerung des zunächst verwendeten reinen Wassers als Auswaschflüssigkeit durch die aerobe Behandlung des Materials 13 im Reaktor 2 erfolgt. Dieser Vorgang entspricht einer Hydrolyse, d. h. einer Auflösung von lösbaren Salzen unter Versäuerung des Wassers.

Die Wirkungsweise der Anlage und insbesondere des Feststoffreaktors 2 geschieht wie folgt:

Das der Einlauföffnung 11 des Reaktors 2 kontinuierlich oder diskontinuierlich zugeführte Material 13 besteht im allgemeinen aus einem nassen organischen Stoff insbesondere aus Biomüll wie eingangs erwähnt. Die Füllhöhe des Materials 13 beträgt ungefähr h_{F} ≈ 2/3 der Innenraumhöhe h, so daß ein oberer Luftraum 16 entsteht. Dabei ist gemäß der Darstellung in Fig. 2 bis 5 die Füllhöhe h_{F} derart bemessen, daß nahezu die gesamte Breite B der Oberfläche 46 des Materials 13 mit Auswaschflüssigkeit 19 besprüht werden kann. Aus diesem Grund wird der obere Bereich des Reaktors zur Vermeidung einer Verkleinerung der Materialoberfläche bei entsprechender Füllhöhe rechteckförmig (Fig. 2), dreieckförmig (Fig. 3) oder insgesamt oval (Fig. 4) im Querschnitt ausgebildet.

Die gleichmäßige Besprühung des Materials 13 durch die Sprüharme 18 mittels der leicht versäuerten Flüssigkeit 19 bewirkt eine Auswaschung von löslichen organischen und/oder anorganischen Substanzen und/oder wasserlöslichen Fettsäuren, die durch Zersetzung des Materials 13 gebildet werden. Dabei ist es von Bedeutung, daß das Material 13 durch eine Art Stachelrührwerk 8 mit paddelförmigen oder schaufelförmigen Umwälzorganen 9 ständig und kontinuierlich oder diskontinuierlich umgewälzt wird, um einerseits eine gute Durchmischung des Materials 13 zu erhalten. Die Umwälzung dient jedoch insbesondere auch dazu, sogenannte vertikale und horizontale Kurzschlußstromkanäle zu vermeiden, die sich durch den Flüssigkeitsstrom der Auswaschflüssigkeit im Material 13 bilden würden, so daß es zu einer ungleichmäßigen Benetzung oder Auswaschung und damit zu toten Zonen kommen würde. Die paddelförmigen oder schaufelförmigen Rührarme 9 können gemäß der Darstellung in Fig. 1 in einer Vielzahl nebeneinander und/oder gegenüberliegend angeordnet sein, wobei die paddelförmigen endseitigen Schaufeln 9 nebeneinanderliegen oder sich überlappen können, so daß es zu einer Art scheibenförmigen Umwälzung des Materials kommt. Aufgrund der Drehbewegung der Welle 7 (Pfeil 47) findet weiterhin ein langsamer Längstransport in Pfeilrichtung 48 durch den Reaktorinnenraum 17 statt, so daß das Material 13 langsam längs durch den Reaktor wandert und schließlich nach einer Verweilzeit von z. B. 4 bis 8 Tagen den Reaktorinnenraum 17 durch die Austragöffnung 14 verläßt.

Während dieser Behandlungsprozedur und insbesondere gleichzeitig oder intermettierend zum Auswaschprozeß wird dem Material 13 über die unteren Kammern 22 gezielt Frischluft 27 zugeführt, was zu einer gleichzeitige aeroben Behandlung des Materials mit einer exotermen Selbsterwärmung des Materials führt. Diese aerobe Behandlung vollzieht sich unter einem mikrobiologischen Abbau der organischen Substanzen mittels entsprechenden Mikroorganismen, wobei die intensive Geruchsentwicklung aufgrund der geschlossenen Bauweise des Reaktors abgekapselt ist. Der obere Luftraum 16 des Reaktors oberhalb des Materials 13 wird mittels einer Saugpumpe 49 über eine Luftaustrittsöffnung 50 ständig abgesaugt, so daß es zu einer ausreichenden Belüftung auch des oberen Luftraums 16 kommt. Durch das gezielte Absaugen der oberen Luft mittels Unterdruck wird sichergestellt, daß nur aerobe Verhältnisse auch im oberen Luftraum 16 vorhanden sind.

Aufgrund des kontinuierlichen oder auch diskontinuierlichen Durchlaufes des Materials 13 über die Längsrichtung des Reaktors befinden sich innerhalb des Reaktors sehr unterschiedliche Materialzusammensetzungen infolge der unterschiedlichen Verweildauer. Beispielsweise befindet sich im Bereich der Einfüllöffnung 11 relativ frisches und unbehandeltes Material, während im Bereich der hinteren Auslaßöffnung 14 das Material bereits z. B. zwischen 4 bis 8 Tage behandelt wurde. Demzufolge kann der Reaktor über seine Länge 1 sehr unterschiedliche Behandlungsstufen umfassen, die stark variieren können.

Dem Reaktor sind nicht näher dargestellte Meßeinrichtungen zur Erfassung der verschiedensten Parameter des Materials wie z. B. seine Zusammensetzung, seine Temperatur, der Feuchtigkeitsgehalt usw. zugeordnet, wobei weiterhin Meßdaten der Auswaschflüssigkeit und der Zuluft zur Verfügung stehen. Je nach Behandlungsfortschritt können dann die nebeneinander geordneten Sprüharme 18 unterschiedlich mit Auswaschflüssigkeit beaufschlagt werden, um eine unterschiedliche Auswaschung der jeweils darunterliegenden Materialzusammensetzung zu erhalten. Gleichermaßen können die z. B. vier unten liegenden Kammern 22 unterschiedlich stark mit Frischluft 27 beaufschlagt werden, um den aeroben Zersetzungsprozeß des Materials 13 zu beeinflussen. Entsprechend kann die durchgeführte Durchmischung des Materials zusätzlich eine Beeinflussung der Materialeigenschaft hervorrufen. Beispielsweise findet bei einem aeroben Abbau des Materials eine gewisse Vergrößerung der Oberfläche des organischen Materials statt, was gleichzeitig den Auswaschprozeß durch die Auswaschflüssigkeit 19 begünstigen würde. Hier muß demzufolge abhängig vom Grad des erfolgten aeroben Abbaus die optimale Menge an Auswaschflüssigkeit zudosiert werden, was empirisch ermittelt werden kann. Weiterhin wirkt sich beispielsweise eine Erhöhung der Temperatur der Auswaschflüssigkeit aufgrund der aeroben Zersetzung des Materials günstig auf die nachfolgenden Behandlungsstufen 3 bis 5 aus, um eine optimale Regenerierung der Auswaschflüssigkeit zu erhalten. Eine bevorzugte Behandlung des Materials im Eingangsbereich sowie im Endbereich des Reaktors ist in den Ansprüchen 3 und 4 beschrieben.

Der Innenraum 17 des Reaktors 2 kann demzufolge mittels entsprechenden Meßeinrichtungen überwacht und der Prozeßfortschritt nach einem vorgegebenen Programm oder auch in Abhängigkeit der Meßwerte geregelt werden.

Die nebeneinanderliegenden unteren Kammern 22 sind mittels den Abdecksieben 23 derart verschlossen, daß das Material 13 weitestgehend nicht in die Kammer 22 gelangen kann. Maßgeblich hierfür ist die Maschenweite mit dem Durchmesser d₁.

Von Zeit zu Zeit muß jedoch das Sieb 23 von Verunreinigungen oder Verstopfungen gereinigt werden, was durch eine Rückspülvorgang erfolgt. Hierfür wird die in der Kammer 22 vorhandene und gesammelte Flüssigkeit 19' entweder durch Schließung der Ventile 25 aufgestaut, so daß es zu einem Flüssigkeitsanstieg innerhalb der Kammer 22 kommt, oder es wird über eine Sammelleitung 51 der Leitung 43, 44 gereinigte Auswaschflüssigkeit entnommen und der Kammer 22 gezielt über Zuführleitungen 52 mit Ventilanordnung 53 zugeführt (siehe Fig. 4, 5). Dabei kann die Flüssigkeit bis zu einem oberen Flüssigkeitsspiegel 33 entsprechend der Höhe der Überlaufwandung 32 aufgestaut werden. Durch eine evtl. zusätzliche Beaufschlagung dieses Flüssigkeitsraums mit Preßluft über den Kompresser 28 kann das jeweils beaufschlagte Sieb 23 frei geblasen und damit gereinigt werden. Dieser Vorgang wird insbesondere auch mittels der Flüssigkeitspumpe 45 oder einer weiteren Pumpe 54 in der Sammelleitung 51 unterstützt.

Der Reinigungsvorgang der Abdecksiebe 23 kann auch derart erfolgen, daß jeweils nur eine Kammer oder nur bestimmte Kammern einem Rückspülvorgang unterzogen werden und die hierdurch aufsteigende Rückspülflüssigkeit in der danebenliegenden oder einer der danebenliegenden Kammern abgeführt wird. So kann beispielsweise die in Fig. 1 erste und dritte Kammer 22 jeweils in der danebenliegenden Kammer beim Rückspülvorgang entleert werden.

Das Rückstauen der Flüssigkeit beispielsweise bis zu einem Flüssigkeitsspiegel 33 kann darüber hinaus auch noch den Effekt haben, daß das Material 13 auf diesem Flüssigkeitspolster auf schwimmt und dieses Material insbesondere bei zähem oder sehr trockenem Material leichter durchmischt werden kann.

Wie zuvor erwähnt, kann das Material 13 unterschiedlichst mit Auswaschflüssigkeit 19 und mit Frischluft 27 bei gleichzeitiger oder intermittierender Umwälzung des Materials behandelt werden. Es werden beispielsweise ca. 0,5 - 2 m³ Auswaschflüssigkeit pro Tag und pro Tonne Material im Reaktor umgesetzt. Die Einschaltdauer des Rührwerks kann zwischen 5 bis 60 min. pro Stunde bei einer Umdrehung von 1 bis 2mal pro Minute betragen.

Fig. 2 zeigt beispielsweise eine Berieselung des Materials über möglichst die gesamte obere Fläche 46 in ihrer maximal möglichen Breite bei gleichzeitiger Umwälzung des Materials mittels des Stachelrührwerks 8. Die durch die Flüssigkeit gleichmäßig hindurchtretende Auswaschflüssigkeit 19 wird im unteren Bereich der Kammer 22 als Flüssigkeit 19' gesammelt. In diesem Zustand findet eine zusätzliche Beaufschlagung mit Frischluft 27 statt, so daß der aerobe Zersetzungsprozeß des Materials 13 gleichzeitig stattfindet.

Im Normalzustand findet gleichzeitig der Auswaschprozeß und die Frischluftzuführung statt. Fig. 3 zeigt eine alleinige Frischluftzufuhr 27 ohne Auswaschvorgang.

Die Darstellung in Fig. 4 zeigt den Rückspülvorgang bis zu einer Flüssigkeitshöhe mit einem Flüssigkeitsspiegel 33 wie zuvor beschrieben. Hierdurch schwimmt das Material auf diesem Flüssigkeitspolster auf, so daß das umlaufende Rührwerk 8 eine leichtere Durchmischung vollziehen kann. Ein geringfügiger Anstieg des Materials innerhalb des Reaktors ist während dieses Mischvorgangs unschädlich.

Die Darstellung der Anordnung in Fig. 2, 3 zeigt den Vorgang der Frischluftzufuhr über die Leitung 31 mit Ventilanordnung 30 über das untere Abdecksieb 23 zum Material 13. Die Luft dient für den aeroben Abbau bzw. die aerobe Zersetzung des Material mittels Mikroorganismen, wobei die Luft im oberen Luftraum 16 gesammelt und über die Luftaustrittsöffnung 50 aus dem Reaktorinnenraum 17 herausgesaugt wird.

Fig. 5 zeigt den Rückspülvorgang über die Leitung 52 mit geöffnetem Ventil 53, bei gleichzeitiger Druckluftbeaufschlagung; Fig. 4 zeigt diesen Vorgang ohne Druckluftbeaufschlagung, d. h. geschlossenem Ventil 30.

Das erfindungsgemäße Verfahren sowie die zugehörige Vorrichtung kann durch die nachfolgend erläuterten Maßnahmen noch weiter optimiert werden.

Wie in Fig. 1 dargestellt, ist im Bereich der Eintrittsöffnung 11 eine zusätzliche Abtrenn- und Tauchwand 55 angebracht. Diese Tauchwand 55 verhindert, daß das eingebrachte Frischmaterial ungehindert auf der Materialoberfläche 46 vom Eintritt 11 quasi im Kurzschluß zum Austritt 14 gelangen kann. Das Frischmaterial muß vielmehr zuerst auf dem durch Pfeil 56 angezeigten Weg unter der Tauchwand 55 hindurch und den unteren Teil des Reaktors durchqueren.

Es kann in speziellen Fällen zweckmäßig sein, daß das im Reaktor behandelte Material vor dem Austritt aus dem Feststoffreaktor 2 hygienisiert wird, d.h. während einer bestimmten Zeit einer bestimmten Temperatur ausgesetzt wird. Dies kann zweckmäßigerweise dadurch geschehen, indem auf eine bestimmte Strecke l₁ ≈ (1/3 - 1/4) 1 der hintere Behälterinhalt 13 zusätzlich erwärmt wird. Hierfür wird beispielsweise der Leitung 43 über eine parallel geschaltete Leitung 57 Umlaufwasser entnommen, welches in einem nicht näher dargestellten Wärmespeicher oder einem schematisch dargestellten Wärmetauscher 58 erhitzt wird. Das Umlaufwasser der Leitung 57 kann dann im oberen Bereich des Reaktors entweder der Sammelleitung 44 gezielt zugegeben werden oder es erfolgt eine gezielte Beaufschlagung z.B. über die Zuleitung 20 im hinteren Reaktorbereich. Dies ist in Fig. 1 schematisch über die letzte Zuleitung 20' dargestellt.

Eine zweckmäßige Weiterbildung des Verfahrens kann auch eine kontinuierliche oder diskontinuierliche Zudosierung von Belebtschlamm bzw. Überschußschlamm aus einer Belebungsanlage in den Bereich der Einfüllöffnung 11 sein. Die mit dem Belebtschlamm eingebrachten aeroben Mikroorganismen stellen einen Reaktionsbeschleuniger zur biochemischen Umsetzung der organischen Materialien dar. Diese Zugabe ist durch Pfeil 59 schematisch angedeutet. Besonders vorteilhaft erweisen sich hierbei bereits an den Abfallstoff adaptierte Mikroorganismen, die entweder der aeroben Perkolatbehandlungsstufe oder beispielsweise einer biologischen Sickerwasser-Reinigungsanlage entnommen werden können. Auch die im Bereich der Auslaßöffnung 14 angedeutete Auftrennung des Materialstroms 15 in einen Teilstrom 15' ermöglicht über die Leitung 60 eine teilweise Rückführung von im Perkolator 6 behandeltem Material. Ein zusätzliches Fördermittel 61 kann diesen Vorgang unterstützen. Durch die teilweise Rückführung von im Perkolator 6 behandeltem Material und den darin enthaltenen bereits adaptierten Mikroorganismen kann ebenfalls eine Reaktionsbeschleunigung bewirkt werden. Dies ist über die Zufuhrleitung 60 mit Pfeil 62 im Bereich der Einfüllöffnung 11 schematisch dargestellt.

Gemäß der Darstellung der Erfindung in den Figuren 2 bis 4 weist das Rührwerk 8 entsprechende Rührarme oder Umwälzorgane auf, die radial von der Antriebswelle 7 wegführen. Gemäß der Darstellung in Fig. 5 und 6 kann zur Unterstützung des Rühreffekts das Rührwerk 8 auch intervallmäßig seine Richtung ändern. Dabei kann der Rühreffekt dadurch verstärkt werden, in dem eine schaufelartige Verdrehung der Rührarme 8 bzw. Umwälzorgane mit einem Anstellwinkel x gemäß Fig. 5 oder auch durch eine bogenförmige Anordnung gemäß der Darstellung in Fig. 6 vorgenommen wird. Dabei ist bei schaufelförmiger Anordnung der Rührarme 8 die Hauptdrehrichtung mit e bezeichnet. Bei der Drehrichtung in Richtung Pfeil e wird eine optimale Untermischung erreicht. Jedoch kann sich bei stark verschmutztem Material 13 beispielsweise mit Plastikfolien und Schnüren usw. eine Umwicklung der Rührarme 8 einstellen. Um die Rührarme 8 von dieser Verschmutzung zu befreien, wird die Rührwelle 7 intervallmäßig in die entgegengesetzte Drehrichtung entsprechend Pfeil f bewegt, wodurch aufgrund der leicht schräg verlaufenden, tangentialen oder bogenförmigen Anordnung der Rührarme 8 die Verschmutzungen durch die Reibung am Material 13 selbst von den Rührarmen 8 abgestreift werden. Der gleiche Effekt ergibt sich sowohl bei der Darstellung nach Fig. 5 als auch nach der Darstellung nach Fig. 6.

Gemäß der Darstellung nach Fig. 6 kann weiterhin das untere Sieb 23 mittels des Rührwerks gereinigt werden. Hierfür kann an den Enden der Rührarme 8 ein Verschleißteil 63 als Schieber angebracht werden. Dieses Verschleißteil bzw. dieser Abstreifer 63 an den Enden des Rührarms 8 kann mit einer nicht näher dargestellten Spannvorrichtung 64 über die zusätzliche Öffnung 65 von außen eingebracht, nachgestellt oder ausgewechselt werden.

Bei einem Behälter 6' in zylindrischer Anordnung gemäß Fig. 6 kann weiterhin das Rührwerk 8 um eine Distanz z aus der Zylinderlängsachse 66 exzentrisch vorzugsweise nach unten verschoben werden. Durch diese Anordnung wird der Rührwerksradius R₂ kleiner als der zylindrische Behälterradius R₁, so daß die Abstreifer 63 dadurch nur auf dem unteren Sieb 23 wirksam sind und nicht einen ungewollten Verschleiß an der Behälterinnenwandung bewirken.

Gemäß dem in Fig. 7 dargestellten Ausschnitt auf die Längsansicht der Rührwelle 7 können die Rührarme 8 vorteilhaft auch um einen Winkel y verdreht auf der Welle 8 angebracht sein. Eine solche Verdrehung der Rührarme bewirkt ähnlich wie eine Schiffsschraube einen Schaufeleffekt und damit einen axialen Vorschub parallel zur Längsachse der Rührwelle 7. Wird beispielsweise die Rührwelle 7 in Pfeilrichtung e verdreht, wird das Material 13 in die Flußrichtung g transportiert. Bei Änderung der Drehrichtung entsprechend dem Pfeil f wird das Material in Richtung des Pfeils h verschoben. Die Drehbewegung der Rührwelle 7 kann demzufolge mit den propellerartig verdrehten Rührarmen einen Vorwärts- oder Rückwärts schub des Materials bewirken.

Bei bestimmten Materialzusammensetzungen wird dieser vorbeschriebene Effekt zur Rückmischung und Animpfung von Frischmaterial 12 mit dem Reaktormaterial 13 benötigt.

Die Erfindung ist nicht auf das dargestellte und beschriebene Ausführungsbeispiel beschränkt. Sie umfaßt auch vielmehr fachmännische Weiterbildungen im Rahmen der Schutzrechtsansprüche.

## Patentansprüche

1. Verfahren zur biologischen Behandlung von organischen Abfällen und insbesondere von biologischen Abfällen (Biomüll) aus dem Haushalt, dem lebensmittelverarbeitenden Gewerbe, der Landwirtschaft oder dergleichen, mit einem Feststoffreaktor (2) in welchem das Material behandelt wird, wobei der Reaktor (2) kontinuierlich oder diskontinuierlich mit Material (13) beschickt wird und die Beschickung an einem Ende und die Entnahme am anderen Ende des Reaktors (2) erfolgt, wobei dem Reaktor (2) weiterhin ein kombiniertes Rühr- und/oder UmwälzWerk (7 bis 10) zugeordnet ist, welches einer Durchmischung des Materials dient, und wobei ein aerober Abbau von organischen Substanzen unter Zufuhr von Frischluft (27) *und Wasser* erfolgt, wobei sich das Material auf Prozeßtemperatur im Reaktor erwärmt, dadurch gekennzeichnet, daß während des aeroben Abbaus des Materials eine kontinuierliche oder diskontinuierliche Auswaschung mittels einer Auswaschflüssigkeit (19) derart erfolgt, daß löslichen, organischen und/oder anorganischen Substanzen sowie ggf. wasserlöslichen Fettsäuren aus dem zu behandelnden Material (13) *in die Auswaschflüssigkeit übernommen und die so belastete* Auswaschflüssigkeit dem unteren Teil des Reaktors (2) entnommen wird, *daß die dem Reaktor (2) entnommene Auswaschflüssigkeit einer Nachbehandlung im Sinne einer Regenerierung unterzogen* und mittels Sprüharmen (18) dem *Reaktor (2)* über die ganze Materialoberfläche *erneut* aufgebracht wird, wobei das Rühr- und/oder Umwälzwerk (7 bis 10) gleichzeitig als Transport-Werk zum Material-Transport dient, bei gleichzeitiger Vermeidung bzw. Zerstörung von Kurzschlußstromkanälen der Auswaschflüssigkeit.

2. Verfahren nach Anspruch 1, dadurch gekennzeichnet, daß das Material (13) durch den Reaktor (2) über seine Länge (1) vorwärts und/oder rückwärts transportierbar ist, wobei die Beaufschlagung des Materials mittels Auswaschflüssigkeit (19) und/oder mittels Frischluft (27) über die Länge (1) des Reaktors vorzugsweise gleichmäßig erfolgt oder je nach Zusammensetzung des Materials variiert.

3. Verfahren nach Anspruch 2, dadurch gekennzeichnet, daß im Einfüllbereich (11) des Reaktors (2) bevorzugt eine gleichmäßige Beaufschlagung des Materials mit Auswaschflüssigkeit (19) und gleichzeitig eine gezielte Frischluftzufuhr (27) erfolgt, wobei der aerobe Abbau des Materials einerseits eine gezielte Erwärmung des Materials (13) und der Auswaschflüssigkeit (19) und die Auswaschflüssigkeit andererseits eine intensive Auswaschung von löslichen organischen und/oder anorganischen Substanzen und/oder wasserlöslichen Fettsäuren bewirkt.

4. Verfahren nach Anspruch 1, dadurch gekennzeichnet, daß im Auslaufbereich mit Auslaßöffnung (14) des Reaktors (2) bevorzugt eine verminderte oder ausgeschaltete Beaufschlagung mit Auswaschflüssigkeit (19) und/oder eine verstärkte Frischluftzufuhr (27) erfolgt, zur Reduzierung des Wassergehalts (Trocknung) des Materials in diesem Bereich, wobei vorzugsweise im Auslaufbereich eine Hygienisierung des Materials mit Zuführung einer vorgewärmten Rücklauf flüssigkeit (19) über ein bestimmtes Zeitintervall erfolgt.

5. Verfahren nach einem der Ansprüche 1 bis 4, dadurch gekennzeichnet, daß die Behandlung des Materials (13) im Reaktor (2) abhängig von seiner organischen Belastung, seiner Temperatur, seinem Wassergehalt durch einen aeroben Abbau und/oder einem Auswaschprozeß von löslichen organischen, anorganischen Stoffen oder wasserlöslichen Fettsäuren unter kontinuierlicher oder diskontinuierlicher Umwälzung des Materials gesteuert und/oder geregelt erfolgt.

6. Verfahren nach einem der Ansprüche 1 bis 5, dadurch gekennzeichnet, daß der Reaktor einen über dem Material (13) liegenden freien Luftraum (16) aufweist, der eine weitestgehende gleichmäßige Berieselung oder Beaufschlagung der maximal möglichen oberen Berieselungsfläche (46) ermöglicht.

7. Verfahren nach einem der Ansprüche 1 bis 6, dadurch gekennzeichnet, daß der Reaktor (2) in seinem unteren Bereich wenigstens zwei Kammern (22) aufweist, die zur Zuführung von Frischluft (27) und/oder als Ablauf der Auswaschflüssigkeit (19') dienen, wobei die Kammern gegenüber dem Reaktorinnenraum (17) über eine Filter- und insbesondere Siebeinrichtung (23) zur Vermeidung des Eintritts von Feststoffen getrennt ist.

8. Verfahren nach Anspruch 7, dadurch gekennzeichnet, daß eine Siebeinrichtung (23) mittels eines Rückspülvorganges in Richtung Reaktorinnenraum (17) durch Fluten der Kammer (22) gereinigt wird, wobei die Rückspülflüssigkeit vorzugsweise einer weiteren und vorzugsweise einer nebengeordneten Kammer zuführbar ist.

9. Verfahren nach Anspruch 8, dadurch gekennzeichnet, daß der Rückspülvorgang durch Lufteinpressung in Richtung Reaktorinnenraum (17) erfolgt.

10. Verfahren nach einem der Ansprüche 7 bis 9, dadurch gekennzeichnet, daß der Reaktor (2) über die Kammer (22) mit Auswaschflüssigkeit (19) derart geflutet wird, daß das Material (13) auf der Flüssigkeit aufschwimmt und daß vorzugsweise das schwimmende Material (13) mittels des Rührwerks (7 bis 10) im Reaktor umgewälzt bzw. vorwärts und/oder rückwärts transportiert wird.

11. Verfahren nach einem der vorhergehenden Ansprüche 1 bis 10, dadurch gekennzeichnet, daß das Rühr- und/oder Umwälzwerk als Stachelrührwerk mit Rührarm (8) und mit einer den Reaktorinnenraum (17) durchsetzenden, horizontalen Welle (7) ausgebildet ist und daß das Material (13) im Sinne einer scheibenförmigen Umwälzung bearbeitet wird, wobei vorzugsweise eine Art scheibenförmiger oder pfropfenförmiger Vorschub des Materials (13) im Reaktorinnenraum (17) erfolgt.

12. Verfahren nach einem der vorhergehenden Ansprüche, dadurch gekennzeichnet, daß die aus dem Reaktor (2) entnommene Auswaschflüssigkeit (19') nachfolgenden Behandlungsstufen (3 bis 5) zugeführt wird, in welcher die Flüssigkeit einem mechanischen und/oder einem anaeroben und/oder einem nachfolgenden aeroben Behandlungsprozeß zur Reinigung und Regenerierung unterworfen ist und daß die so behandelte Auswaschflüssigkeit vorzugsweise zur Rückspülung der Kammern (22) und/oder dem über dem Material (13) liegenden freien Raum (16) im Reaktor (6) zugeführt wird.

13. Verfahren nach Anspruch 12, dadurch gekennzeichnet, daß die Behandlungsstufen (3 bis 5) einen Störstoffabscheider (3) zur Abscheidung von Sinkstoffen wie Sand, Steine oder dergleichen umfassen, in welchem schwimmende Schwebstoffe, wie Kunststoff, Holz oder dergleichen austragbar sind, daß vorzugsweise weiterhin die Behandlungsstufen (3 bis 5) einen nachfolgenden anaeroben Metanreaktor (4) zur Behandlung der Auswaschflüssigkeit umfassen, wobei die mit Organik angereicherte Auswaschflüssigkeit durch Metanbakterien abgebaut und gereinigt wird unter Bildung von Biogas (40) als Stoffwechselprodukt und daß die so gereinigte Auswaschflüssigkeit vorzugsweise einer nachfolgenden aeroben Reinigungsstufe (5) zugeführt wird, in welcher ein aerober Zustand herstellbar ist und daß die so gereinigte und regenerierte Auswaschflüssigkeit dem Reaktor (2) wieder zuführbar ist.

14. Verfahren nach Anspruch 13, dadurch gekennzeichnet, daß zur Reaktionsbeschleunigung in den Einlaufbereich (11) Belebtschlamm einer aeroben Belebungsanlage (59) zudosiert wird.

15. Verfahren nach Anspruch 14, dadurch gekennzeichnet, daß zur Reaktionsbeschleunigung eine Teilmenge (15) von behandeltem Material (13) aus dem Auslauf (14) zurückgeführt und in den Einlaufbereich (11) zudosiert (62) wird.

16. Vorrichtung zur biologischen Behandlung von organischen Abfällen und insbesondere von biologischen Abfällen (Biomüll) aus dem Haushalt, dem lebensmittelverarbeitenden Gewerbe, der Landwirtschaft oder dergleichen, nach einem oder mehreren der vorhergehenden Ansprüche, mit einem Feststoffreaktor (2), dessen Einfüllöffnung (11) sowie Auslaßöffnung (14) für einzubringendes Material diametral gegenüberliegend angeordnet sind, dadurch gekennzeichnet, daß der Reaktor (2) von einer horizontalen, reversierbar umlaufenden Welle (7) für ein umlaufendes Rühr-, Durchmisch- und Transportwerk (8 bis 10), in Längs- bzw. Materialtransportrichtung durchsetzt ist und daß im Reaktorinnenraum (17) über seine Länge 1 verteilte Sprüharze (18) für Auswaschflüssigkeit (19) vorgesehen sind, wobei Kammern (22) im Bodenbereich des Reaktors (2) zur Aufnahme der Flüssigkeit (19) und/oder zur Frischluftzuführung (27) vorgesehen sind.

17. Vorrichtung nach Anspruch 16, dadurch gekennzeichnet, daß das Rührwerk als Stachelrührwerk (8) mit paddelförmigen oder schaufelförmigen Rührarmen oder Umwälzorganen (8, 9) ausgebildet ist, wobei Rührarme (8, 9) zur Erzeugung einer vorwärts oder rückwärts gerichteten Bewegung vorzugsweise propellerartig verdrehbar sind.

18. Vorrichtung nach Anspruch 16, dadurch gekennzeichnet, daß die paddelförmigen oder schaufelförmigen Umwälzorgane (9) an einer Welle (7) gegenüberliegend und/oder sich überlappend nebeneinanderliegend angeordnet sind.

19. Vorrichtung nach einem der Ansprüche 16 bis 18, dadurch gekennzeichnet, daß die Arme des Rührwerks aus einer radialen Symmetrieachse um einen Winkel x versetzt und insbesondere schräg angeordnet sind oder einen gebogenen Verlauf aufweisen.

20. Vorrichtung nach Anspruch 16, dadurch gekennzeichnet, daß zur Rückführung von behandeltem Material (13) über eine Förderleitung (60) zum Einlauf (11) eine Fördereinrichtung (61) vorgesehen ist.

## Claims

1. Process for the biological treatment of organic waste materials and in particular of biological waste materials (biowaste) from domestic households, the food processing industry, agriculture or the like, with a solid waste reactor (2), in which the material is processed, whereby the reactor (2) is continuously or intermittently charged with material (13) and material is charged at one end and removed at the other end of the reactor (2), whereby a combined agitator and/or circulation unit (7 to 10) is additionally allocated to the reactor (2) serving to thoroughly mix the material, and whereby aerobic degradation of organic substances occurs by adding fresh air (27) and water, whereby the temperature of the material increases to process temperature in the reactor,
characterised in that during the aerobic degradation of the material a continuous or intermittent washout is conducted by means of a washing liquid (19) such that soluble, organic and/or inorganic substances and also possibly water-soluble fatty acids are transferred into the washing liquid from the material (13) to be treated and the thus charged washing liquid is removed from the lower portion of the reactor (2), that the washing liquid removed from the reactor (2) is subjected to a subsequent treatment in the sense of a regeneration and is once again fed to the reactor (2) and applied over the entire surface of the material by means of spraying arms (18), whereby the agitator and/or circulation unit (7 to 10) at the same time serves as a transport unit for transporting the material, while at the same time preventing or destroying bypass channels of the washing liquid.

2. Process according to Claim 1, characterised in that the material (13) may be transported forwards and/or backwards through the reactor (2) over its length (1), whereby washing liquid (19) and/or fresh air (27) are preferably evenly subjected to the material over the length (1) of the reactor or this application is varied according to the composition of the material.

3. Process according to Claim 2, characterised in that in the charging region (11) of the reactor (2) the material is preferably subjected evenly to washing liquid (19) and a determined supply of fresh air (27) is achieved at the same time, whereby the aerobic degradation of the material firstly causes a determined warming of the material (13) and the washing liquid (19) and the washing liquid secondly effects an intensive washout of soluble organic and/or inorganic substances and/or water-soluble fatty acids.

4. Process according to Claim 1, characterised in that in the discharge region with outlet (14) of the reactor (2) a reduced or excluded application of washing liquid (19) and/or an increased supply of fresh air (27) occurs for reduction of the water content (drying) of the material in this region, whereby the material is sanitised preferably in the discharge region with the supply of preheated return liquid (19) over a specific period of time.

5. Process according to one of Claims 1 to 4, characterised in that the treatment of the material (13) in the reactor (2) is conducted in a controlled and/or regulated manner, depending on its organic content, its temperature, its water content, by means of an aerobic degradation and/or a washout process of soluble organic, inorganic substances or water-soluble fatty acids with continuous or intermittent circulation of the material.

6. Process according to one of Claims 1 to 5, characterised in that the reactor has a free air space (16) lying above the material (13) to allow the maximum possible upper spraying surface (46) to be sprayed or covered as evenly as possible.

7. Process according to one of Claims 1 to 6, characterised in that the reactor (2) has at least two chambers (22) in its lower region which serve to supply fresh air (27) and/or as discharge for the washing liquid (19'), whereby the chambers are separated from the interior (17) of the reactor via a filter, and in particular screening means (23) to prevent solid materials from entering.

8. Process according to Claim 7, characterised in that a screening means (23) is cleaned by means of a backwash process in the direction of the reactor interior (17) by flooding the chamber (22), whereby the backwash liquid may preferably be supplied to a further, and preferably secondary chamber.

9. Process according to Claim 8, characterised in that the backwash process is conducted by injecting air in the direction of the reactor interior (17).

10. Process according to one of Claims 7 to 9, characterised in that the reactor (2) is flooded with washing liquid (19) via the chamber (22) in such a manner that the material (13) floats on the liquid, and that preferably the floating material (13) is circulated or transported forwards and/or backwards in the reactor by means of the agitator unit (7 to 10).

11. Process according to one of the preceding Claims 1 to 10, characterised in that the agitator and/or circulation unit is constructed as a toothed agitator means with agitator arm (8) and with a horizontal shaft (7) passing through the reactor interior (17), and that the material (13) is processed in the sense of a slice-type circulation, whereby preferably a slice-type or slug-type forward feed of the material (13) is achieved in the reactor interior (17).

12. Process according to one of the preceding claims, characterised in that the washing liquid (19') removed from the reactor (2) is fed to subsequent treatment stages (3 to 5), in which the liquid is subjected to a mechanical and/or an anaerobic and/or a subsequent aerobic treatment process for cleaning and regeneration, and that the thus treated washing liquid is preferably supplied for backwashing the chambers (22) and/or to the free space (16) lying above the material (13) in the reactor (6).

13. Process according to Claim 12, characterised in that the treatment stages (3 to 5) comprise a contaminant separator (3) for the separation of settling materials such as sand, stones or the like, in which separator floating suspended materials such as plastic, wood or the like may be discharged, that preferably the treatment stages (3 to 5) additionally comprise a downstream anaerobic methane reactor (4) for treatment of the washing liquid, whereby the washing liquid containing organic substance is degraded by methane bacteria and cleaned upon the formation of biogas (40) as metabolic product, and that the thus cleaned washing liquid is preferably fed to a subsequent aerobic cleaning stage (5), in which an aerobic state may be produced, and that the thus cleaned and regenerated washing liquid may be fed to the reactor (2) again.

14. Process according to Claim 13, characterised in that to accelerate the reaction, activated sludge from an aerobic activated sludge unit (59) is added in metered doses into the filling region (11).

15. Process according to Claim 14, characterised in that to accelerate the reaction, a portion (15) of treated material is returned from the outlet (14) and is added in metered doses (62) into the filling region (11).

16. Device for the biological treatment of organic waste materials and in particular of biological waste materials (biowaste) from domestic households, the food processing industry, agriculture or the like, according to one or more of the preceding claims, with a solid waste reactor (2), of which the filling opening (11) and outlet (14) for material (13) to be fed in are diametrically opposed, characterised in that the reactor (2) has a horizontal reversible shaft (7) for a rotating agitator, mixer and transport unit (8 to 10) passing through it in longitudinal direction or in the transport direction of the material, and that spraying arms (18) distributed in the interior (17) of the reactor over its length l are provided for washing liquid, whereby chambers (22) are provided in the base region of the reactor (2) to receive the liquid (19) and/or for the supply of fresh air (27).

17. Device according to Claim 16, characterised in that the agitator unit is constructed as a toothed agitator unit (8) with paddle- or blade-type agitator arms or circulation members (8, 9), whereby agitator arms (8, 9) may be relatively rotated preferably in the manner of a propeller to generate a forward- or backward-directed movement.

18. Device according to Claim 16, characterised in that the paddle- or blade-type circulation members (9) are arranged on a shaft (7) to lie opposite and/or overlap next to one another.

19. Device according to one of Claims 16 to 18, characterised in that the arms of the agitator unit are displaced by an angle x from a radial axis of symmetry and in particular are arranged on a slope or have a curved course.

20. Device according to Claim 16, characterised in that a transport means (61) is provided to return treated material (13) via a transport pipe (60) to the inlet (11).

## Revendications

1. Procédé de traitement biologique de déchets organiques, et en particulier de déchets biologiques (bio-ordures) d'origine domestique, de l'industrie de transformation des produits alimentaires, de l'agriculture ou analogue, avec un réacteur pour matériaux solides (2) dans lequel le matériau est traité, le réacteur (2) étant alimenté en continu ou en discontinu par du matériau (13), l'alimentation s'effectuant à une extrémité, le prélèvement à l'autre extrémité de réacteur (2), un dispositif d'agitation et/ou de mise en circulation (7 à 10) combiné étant en outre associé au réacteur (2) et servant à effectuer un mélange intime du matériau, une décomposition aérobie des substances organiques avec apport d'air frais (27) et d'eau étant effectuée, le matériau étant chauffé dans le réacteur à la température du processus,
caractérisé en ce que
• pendant la décomposition aérobie du matériau, est effectué une extraction par lavage continu ou discontinu, au moyen d'un liquide d'extraction par lavage (19),
• les substances solubles, organiques et non-organiques ainsi que, le cas échéant, les acides gras solubles dans l'eau sont transférés du matériau (13) à traiter et passés dans le liquide d'extraction par lavage, et le liquide d'extraction par lavage ainsi chargé est prélevé de la partie inférieure du réacteur (2),
• le liquide d'extraction par lavage prélevé du réacteur (2) est soumis à un retraitement dans le sens d'une régénération et est appliqué de nouveau, au moyen de bras de pulvérisation (18), dans le réacteur (2) sur toute la surface de matériau, le dispositif d'agitation et/ou de mise en circulation (7 à 10) servant simultanément de dispositif de transport pour assurer le transport du matériau, en évitant et/ou en détruisant simultanément la formation de canaux de court-circuit pour le liquide d'extraction par lavage.

2. Procédé selon la revendication 1,
caractérisé en ce que
le matériau (13) est susceptible d'être transporté dans réacteur (2), par déplacement en avant ou en arrière sur sa longueur (1), l'exposition du matériau à du liquide d'extraction par lavage (19) et/ou à de l'air frais (27) sur la longueur (1) du réacteur s'effectuant, de préférence, régulièrement ou étant soumise à une variation, selon la composition du matériau.

3. Procédé selon la revendication 1,
caractérisé en ce que,
dans la zone d'introduction (11) du réacteur (2), s'effectue de préférence une exposition régulière du matériau au liquide d'extraction par lavage (19) est simultanément un apport d'air frais (27) fait à dessein, la décomposition aérobie du matériau, d'une part, provoquant un échauffement à dessein du matériau (13) et du liquide d'extraction par lavage (19) et le liquide d'extraction par lavage, d'autre part, provoquant une évacuation par lavage intense des substances solubles organiques et/ou non-organiques et/ou des acides gras solubles dans l'eau.

4. Procédé selon la revendication 1,
caractérisé en ce que
dans la zone d'évacuation dotée de l'ouverture d'évacuation (14) du réacteur (2), s'effectue de préférence une exposition diminuée ou stoppée au liquide d'extraction par lavage (19) et/ou un apport d'air frais (27) amplifié, dans le but de réduire la teneur en eau (séchage) du matériau dans cette zone, de préférence dans la zone d'évacuation une hygiénisation du matériau et réalisée avec amenée d'un liquide de retour (19) préchauffé sur un intervalle de temps déterminé.

5. Procédé selon l'une des revendications 1 à 4,
caractérisé en ce que
le traitement du matériau (13) dans le réacteur (2) s'effectue en fonction de sa charge organique, de sa température, de sa teneur en eau, par une décomposition aérobie et/ou par un processus d'extraction par lavage des substances solubles, organiques, non organiques, ou des acides gras solubles dans l'eau, avec mise en circulation continue ou discontinue du matériau, de façon commandée et/ou régulée.

6. Procédé selon l'une des revendications 1 à 5, caractérisé en ce que
le réacteur présente une enceinte à air (16) libre située au-dessus du matériau (13) qui permet d'arroser ou d'exposer de façon aussi régulière que possible la surface d'arrosage (46) supérieure maximale possible.

7. Procédé selon l'une des revendications 1 à 6,
caractérisé en ce que
le réacteur (2) présente dans sa zone inférieure au moins deux chambres (22) qui sert à l'amenée d'air frais (27) et/ou d'évacuation du liquide d'extraction par lavage (19'), les chambres étant séparées de l'espace intérieur de réacteur (17) par l'intermédiaire d'un dispositif de filtration et, en particulier, de tamisage (23), dans le but d'éviter toute pénétration de solides.

8. Procédé selon la revendication 7,
caractérisé en ce qu'
un dispositif de tamisage (23) est nettoyé au moyen d'un processus à rinçage à contre-courant dans la direction de l'espace intérieur de réacteur (17) par inondation de la chambre (22), le liquide de rinçage à contre-courant pouvant être amené de préférence à une autre chambre et de préférence à une chambre disposée à côté.

9. Procédé selon la revendication 8,
caractérisé en ce que
le processus de rinçage à contre-courant s'effectue par insufflation d'air sous pression, dans la direction de l'espace intérieur de réacteur (17).

10. Procédé selon l'une des revendications 7 à 9,
caractérisé en ce que
le réacteur (2) est inondé par la chambre (22) avec du liquide d'extraction par lavage (19), de manière que le matériau (13) soit mis en flottation sur le liquide et que, de préférence, le matériau flottant (13) soit mis en circulation, respectivement transporté dans le sens avant et/ou dans le sens arrière dans le réacteur, au moyen du dispositif agitateur (7 à 10).

11. Procédé selon l'une des revendications 1 à 10 précédentes,
caractérisé en ce que
le dispositif d'agitation et/ou de mise en circulation est réalisé sous la forme de dispositif agitateur à ergots, équipé d'un bras agitateur (8) et d'un arbre (7) horizontal, traversant l'espace intérieur de réacteur (17), et en ce que le matériau (13) est travaillé dans le sens d'une mise en circulation en forme de disque, de préférence un type de progression en forme de disque ou en forme de bouchon du matériau (13) dans l'espace intérieur de réacteur (17) étant effectuée.

12. Procédé selon l'une des revendications précédentes,
caractérisé en ce que
le liquide d'extraction par lavage (19') prélevé du réacteur (2) est amené à des étages de traitement (3 à 5) subséquents, dans lesquels le liquide est soumis à un processus de traitement de nature mécanique et/ou anaérobie et/ou aérobie subséquent pour épurer ou régénérer, et en ce que le liquide d'extraction par lavage ainsi traité est amené de préférence pour un rinçage à contre-courant des chambres (22) et/ou à l'enceinte libre (16) située au-dessus du matériau (13) dans le réacteur (6).

13. Procédé selon la revendication 12,
caractérisé en ce que
• les étages de traitement (3 à 5) comprennent un séparateur à substances parasites (3) destiné à séparer les substances non-flottantes telles que le sable, les pierres ou analogues, séparateur dans lequel les substances flottantes mises en flottaison, telle que la matière plastique, le bois, ou analogues, peuvent être extraites,
• de préférence, en plus, les étages de traitement (3 à 5) comprennent un réacteur à méthane (4), anaérobie, monté en aval, pour le traitement du liquide d'extraction par lavage, le liquide d'extraction par lavage ayant été enrichi en produits organiques étant décomposé et épuré par des bactéries méthanogènes avec formation de biogaz (40) à titre de produit du métabolisme,
• le liquide d'extraction par lavage, ainsi épuré, est amené de préférence à un étage d'épuration (5) aérobie subséquent, dans lequel peut être constitué un état aérobie, et
• le liquide d'extraction par lavage ainsi épuré et régénéré peut être ramené de nouveau au réacteur (2).

14. Procédé selon la revendication 13,
caractérisé en ce que
pour accélérer la réaction, on amène de façon dosée dans la zone d'introduction (11), de la boue activée venant d'une installation d'activation aérobie (59).

15. Procédé selon la revendication 14,
caractérisé en ce que
pour accélérer la réaction, une quantité partielle (15) de matériau traité (13) est retournée de l'évacuation (14) et amenée de façon dosée (62) dans la zone d'introduction (11).

16. Dispositif de traitement biologique de déchets organiques et, en particulier, de déchets biologiques (bio-ordures) d'origine domestique, de l'industrie de transformation des produits alimentaires, de l'agriculture ou analogues, selon une ou plusieurs des revendications précédentes, avec un réacteur à solides (2), dont l'ouverture d'introduction (11) ainsi que l'ouverture d'évacuation (14) sont disposées diamétralement opposées pour le matériau (13) à introduire,
caractérisé en ce que
• le réacteur (2) est traversé, dans la direction longitudinale et/ou de transport du matériau, par un arbre (7) mis en mouvement, de façon réversible, horizontale, destiné à un dispositif d'agitation, de mélange intime et de transport (8 à 10) mis en mouvement, et,
• dans l'espace intérieur de réacteur (17) sont prévus, répartis sur sa longueur 1, des bras de pulvérisation ou aspersion (18) destinés au liquide d'extraction par lavage (19), des chambres (22) étant prévues dans la zone de fond du réacteur (2) pour recevoir le liquide (19) et/ou pour l'amenée d'air frais (27).

17. Dispositif selon la revendication 16,
caractérisé en ce que
le dispositif agitateur est réalisé sous la forme de dispositif agitateur à ergots (8) avec des bras agitateurs ou des organes de mise en circulation (8, 9) en forme de palettes et/ou d'aubes, les bras agitateurs (8, 9) étant susceptibles de tourner, de préférence à la façon d'une hélice, en vue de générer un mouvement dirigé vers l'avant ou vers l'arrière.

18. Dispositif selon la revendication 16,
caractérisé en ce que
les organes de mise en circulation (9) en forme de palettes ou en forme d'aubes sont disposés sur un arbre (7), en position opposée et/ou les uns à côté des autres en se chevauchant.

19. Dispositif selon l'une des revendications 16 à 18,
caractérisé en ce que
les bras du dispositif agitateur sont décalés d'un angle x par rapport à un axe de symétrie radial et, en particulier, sont disposés obliquement ou ont une allure bombée.

20. Dispositif selon la revendication 16,
caractérisé en ce qu'
un dispositif de transport (61) est prévu pour assurer le recyclage du matériau (13) traité, par une conduite de transport (60) allant à l'entrée (11).
